# FASCICULE DE BREVET EUROPEEN

(11) **EP 3 634 535 B1**
(45) Date de publication et mention de la délivrance du brevet: **01.12.2021**
(21) Numéro de dépôt: 18735362.8
(22) Date de dépôt: 08.06.2018
(51) Int. Cl.: A61M 5/00, B65D 25/10

(54) **PLATEAU POUR LE MAINTIEN DE TUBES DE DISPOSITIF D'INJECTION DE MÉDICAMENTS**
PLATTE ZUM HALTEN VON SCHLÄUCHEN EINER ARZNEIMITTELINJEKTIONSVORRICHTUNG
PLATE FOR HOLDING DRUG INJECTION DEVICE TUBES

(30) Priorité: 09.06.2017 FR 1755198
(43) Date de publication de la demande: 15.04.2020
(73) Titulaire: Crossject, 21000 Dijon (FR)
(72) Inventeur: VIVIEN, Gilles, 92240 Malakof (FR); VIGOT, Xavier, 21260 Veronnes (FR); LABROT, Magalie, 39700 Amange (FR)
(74) Mandataire: Germain Maureau
(86) Numéro de dépôt international: PCT/FR2018/051343
(87) Numéro de publication internationale: WO 2018/224794

(56) Documents cités:
- US-A1- 2014 034 545

## Description

La présente invention concerne un plateau de maintien de tubes de dispositif d'injection de médicaments, ainsi que des procédés de traitement de tubes mettant en œuvre un tel plateau de maintien.

Un type de dispositif d'injection connu, présenté notamment par le document FR-A1-3038232, réalise sans aiguille des injections intradermiques, sous-cutanées ou intramusculaires, de principes actifs contenus dans un fluide à usage thérapeutique en médecine pour l'homme ou en médecine vétérinaire. Le fluide peut être un gel ou un liquide plus ou moins visqueux.

Ces dispositifs à usage unique contiennent une source d'énergie comme un générateur de gaz sous pression, délivrant un gaz brutalement libéré sur un piston ajusté dans un cylindre formé par un tube en verre, pour propulser le fluide contenu sous ce piston vers une buse d'injection en contact avec la peau, et l'injecter en dessous de cette peau.

Le tube en verre présentant une collerette à chaque extrémité, subit lors de sa préparation un cycle complet réalisé dans un environnement à air contrôlé, comprenant successivement un lavage, un séchage, un dépôt de silicone, une dépyrogénation et une stérilisation finale.

Par ailleurs les seringues utilisées habituellement pour une injection avec aiguille, comprenant un piston poussé par un opérateur, comportent généralement un tube présentant une collerette à une seule extrémité. Pour le cycle de préparation de ces tubes, on les insère dans des perçages circulaires de maintien formés sur un plateau, leurs collerettes formant des rebords venant en appui au-dessus des perçages, et maintenant ces tubes verticalement.

De cette manière un ensemble de tubes disposés dans un même plateau est facilement manipulé dans différents appareils pour réaliser la succession des opérations de préparation. La manipulation des tubes se fait de manière simple, rapide et sécurisée.

Toutefois pour des tubes, notamment en verre, comportant une collerette à chaque extrémité présentant des diamètres similaires, on ne peut pas utiliser ce type de plateau car les perçages circulaires de maintien présentant un diamètre suffisant pour recevoir la collerette inférieure du tube, ne pourraient alors pas retenir la collerette supérieure. Un tableau qui permet l'insertion des tubes comportant une collerette à chaque extrémité d'une certaine manière est connu du document US 2014/034545 A1.

La présente invention est définie dans la revendication 1 et a notamment pour but d'éviter ces inconvénients de la technique antérieure.

Elle propose à cet effet un plateau prévu pour maintenir des tubes de dispositif d'injection comportant une collerette à chaque extrémité d'un corps, afin de réaliser des opérations de préparation de ces tubes, ce plateau comprenant des perçages prévus pour recevoir chacun le corps du tube, étant remarquable en ce que chaque perçage comporte une partie de maintien ne laissant pas passer la collerette supérieure du tube, qui se prolonge latéralement par une partie d'introduction permettant le passage de la collerette inférieure.

Un avantage de ce plateau est que de manière simple, rapide et efficace, un opérateur peut introduire une collerette de chaque tube dans la partie d'introduction, puis faire glisser latéralement ce tube vers la partie de maintien. Le tube est alors maintenu verticalement par sa collerette supérieure retenue au-dessus du perçage.

Ainsi, chaque perçage comporte une partie de maintien configurée pour ne pas laisser passer la collerette supérieure du tube, ladite partie de maintien se prolongeant latéralement par une partie d'introduction configurée pour permettre le passage de la collerette inférieure du tube.

Le plateau de maintien des tubes selon l'invention peut comporter de plus une ou plusieurs des caractéristiques suivantes, qui peuvent être combinées entre elles.

Avantageusement, chaque perçage comporte d'un côté avant la partie de maintien présentant vers l'avant un contour en arc de cercle prévu pour recevoir de manière ajustée le corps du tube.

Dans ce cas, avantageusement la partie de maintien se prolonge vers l'arrière par la partie d'introduction comportant un contour en arc de cercle prévu pour permettre le passage de la collerette inférieure du tube. On obtient de cette manière un perçage présentant une surface minimum pour préserver la rigidité de la plaque, permettant à la fois l'introduction du tube et son maintien.

Avantageusement, la partie de maintien comporte vers l'avant une paroi verticale. Cette paroi augmente le maintien vertical des tubes dans la plaque.

Avantageusement, le plateau comporte des éléments élastiques disposés entre la partie de maintien et la partie d'introduction. Ces éléments élastiques permettent un clipsage du tube dans la partie de maintien, assurant son positionnement dans cette partie.

En particulier, les éléments élastiques peuvent former des languettes comportant chacune un bossage séparant la partie de maintien de la partie d'introduction.

Avantageusement, le plateau comporte une matière plastique comprenant des polymères à cristaux liquides, mise en œuvre par injection. Cette matière plastique présente à la fois une bonne rigidité, une tenue aux différents agents chimiques et une tenue aux températures élevées de stérilisation sèche.

L'invention a aussi pour objet un procédé de traitement de tubes contenus dans un plateau comprenant l'une quelconque des caractéristiques précédentes, qui enduit au préalable les tubes de silicone, puis stérilise le plateau supportant les tubes par un procédé de chaleur sèche.

L'invention a de plus pour objet un procédé de traitement de tubes contenus dans un plateau comprenant l'une quelconque des caractéristiques précédentes, qui dispose le plateau supportant les tubes dans un support normalisé, puis dans un sachet scellé formé dans un polyéthylène haute densité, avant un cycle de stérilisation.

L'invention a de plus pour objet un procédé de traitement de tubes contenus dans un plateau comprenant l'une quelconque des caractéristiques précédentes, qui comporte une étape finale de conditionnement du plateau supportant les tubes, réalisée suivant la norme française standard « NF ISO 11040-7 ».

L'invention sera mieux comprise et d'autres caractéristiques et avantages apparaîtront plus clairement à la lecture de la description ci-après donnée à titre d'exemple, en référence aux dessins annexés dans lesquels :
- la figure 1 présente un tube en verre d'un dispositif d'injection sans aiguille, comportant une collerette de chaque côté ;
- la figure 2 présente un plateau avec perçage selon l'invention, prévu pour recevoir ces tubes en verre ;
- la figure 3 est une vue de détail de ce plateau ;
- la figure 4 est une vue de dessus d'un perçage de ce plateau ; et
- la figure 5 est une vue d'ensemble de ce plateau ajusté dans un contour prévu pour un conditionnement normalisé.

Pour plus de clarté, les éléments identiques ou similaires sont repérés par des signes de référence identiques sur l'ensemble des figures.

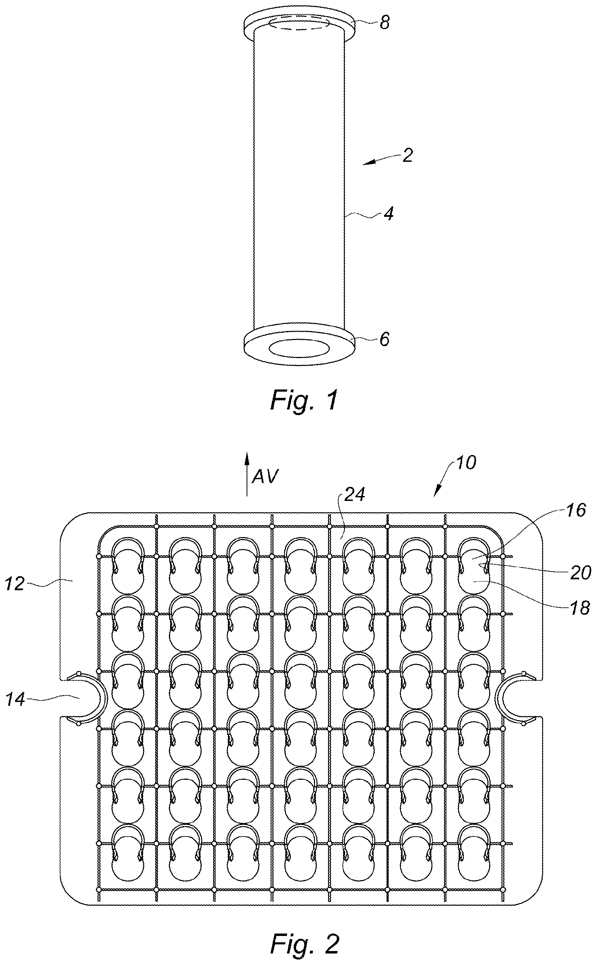

La figure 1 présente un tube en verre 2 de dispositif d'injection, comportant un corps cylindrique 4 présentant une collerette inférieure 6 et une collerette supérieure 8, formant chacune un bourrelet extérieur de révolution autour de l'axe principal du corps.

Le tube en verre 2 peut être utilisé en particulier par le dispositif d'injection présenté ci-dessus et illustré par le document de l'art antérieur FR-A1-3038232.

Les figures 2, 3 et 4 présentent un plateau 10 de forme rectangulaire, comprenant dans une direction transversale un côté appelé par convention côté avant, indiqué par la flèche « AV ».

Le plateau 10 comporte une plaque plane 24 présentant un contour plat 12 comprenant au milieu des deux côtés perpendiculaires à la direction avant, une échancrure 14 facilitant la manipulation de ce plateau.

Le plateau 10 comporte suivant la direction avant-arrière sept rangées identiques de six perçages, chacun de ces perçages étant prévu pour recevoir un tube de verre 2 qui est maintenu en présentant son axe perpendiculaire à ce plateau.

Un réseau de nervures 26 entoure les perçages pour rigidifier la plaque plane 24.

Chaque perçage comporte une partie avant de maintien 16 présentant vers l'avant un contour en demi-cercle comprenant une paroi verticale 22 dépassant au-dessus de la plaque 24, qui est sensiblement ajustée autour du corps 4 d'un tube de verre 2.

La collerette supérieure 8 du tube de verre 2 repose sur le dessus de la paroi verticale 22, la hauteur de cette paroi sensiblement égale au diamètre du corps 4, assurant un maintien vertical de ce tube.

La partie avant de maintien 16 de chaque perçage se prolonge vers l'arrière par deux côtés parallèles droits, qui débouchent dans une partie arrière d'introduction 18 comportant un contour circulaire présentant un diamètre un peu supérieur à celui de la collerette inférieure 6.

A la hauteur de la plaque plane 24, la paroi verticale 22 se prolonge vers l'arrière, au niveau de chaque côté parallèle droit reliant la partie avant de maintien 16 à la partie arrière d'introduction 18, par une languette élastique 20 venant de cette paroi verticale. Chaque languette élastique 20 présente une courbure qui suit d'abord le contour circulaire de la partie avant de maintien 16, puis après un bossage central 28 tourné vers l'intérieur, qui suit le contour circulaire de la partie arrière d'introduction 18.

La distance minimum entre les deux bossages 28 des languettes élastiques 20 est un peu inférieure au diamètre du corps 4 du tube de verre 2.

Après avoir introduit la collerette inférieure 6 d'un tube de verre 2 dans la partie arrière d'introduction 18, on presse vers l'avant ce tube, son corps 4 s'insérant entre les deux languettes 20 pour les écarter par déformation élastique en pressant sur leur bossage central 28.

Après avoir passé les bossages des languettes 20, le tube de verre 2 est repoussé par ces languettes vers l'avant pour venir s'ajuster au fond de la partie avant de maintien 16, contre la paroi verticale 22.

On obtient à la fin un maintien vertical de chaque tube de verre 2 grâce à la collerette supérieure 8 posée au-dessus de la paroi verticale 22, et aux deux languettes 20 qui pressent en permanence sur le corps 4 vers l'avant pour le plaquer sur cette paroi verticale.

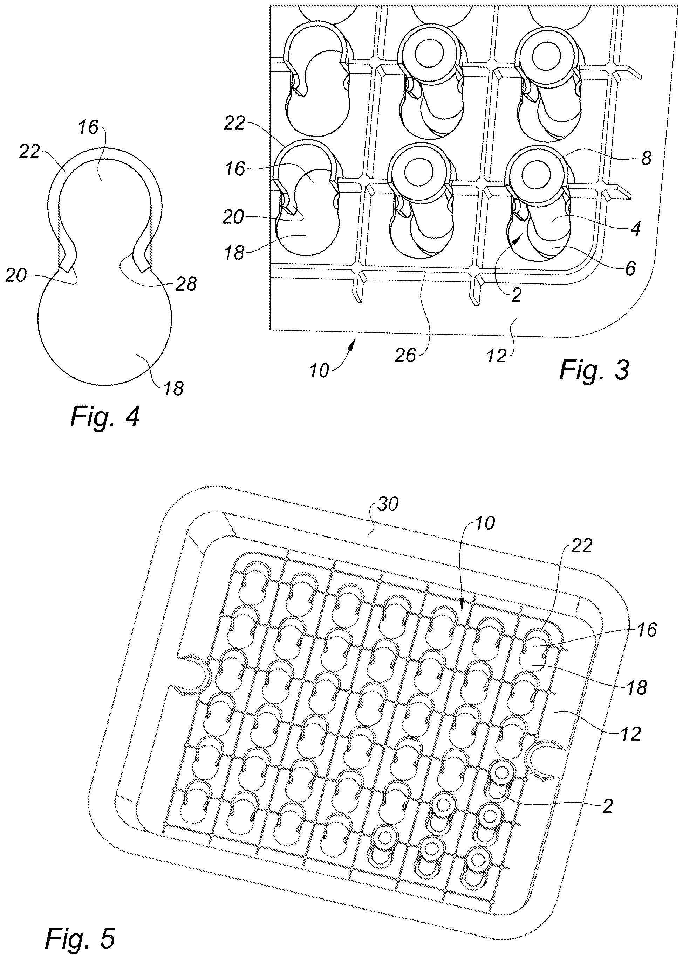

La figure 5 présente le plateau 10 ajusté dans un support normalisé 30 formant un contour, appelé aussi «Tub», permettant un positionnement dans différents appareils de traitement des tubes de verre 2. On réalise ensuite une mise en sachet du plateau complet 10 avec son support 30, recevant un ensemble de tubes en verre 2, et un scellement de ce sachet pour réaliser une stérilisation des tubes.

Avantageusement on utilise des sachets en matière plastique utilisant un polyéthylène haute densité, qui peut en particulier être un matériau commercialisé sous la marque déposée « Tyvek », permettant un passage réduit d'humidité, une bonne conduction de la chaleur, une forte barrière microbienne, et une résistance mécanique élevée, en particulier pour la tenue aux perforations.

Le conditionnement final est avantageusement réalisé suivant la norme française standard « NF ISO 11040-7 », spécifiant l'emballage de systèmes pour délivrer des tubes stérilisés prêts à être remplis.

Avantageusement on réalise après le lavage des tubes 2 une enduction de silicone, suivie d'une stérilisation par un procédé de réduction des pyrogènes comprenant un chauffage à la chaleur sèche pour obtenir une forte réduction des endotoxines qui sont des pyrogènes.

En particulier un cycle de chauffage en chaleur sèche à 240°C a pour avantage de réaliser en même temps une réticulation de la silicone, ce qui évite par la suite des rejets de cette silicone dans le médicament après le remplissage du tube en verre 2, et pendant l'utilisation finale du dispositif d'injection sur le patient. De plus ce procédé évite une stérilisation à l'oxyde d'éthylène qui introduit des matières toxiques.

Avantageusement le plateau 10 est réalisé par moulage par injection d'une matière plastique comprenant des polymères à cristaux liquides, appelée « LCP » (acronyme des termes anglais « Liquid Crystal Polymer »), qui sont des matériaux à haute performance mécanique résistant au cycle de chaleur sèche à 240°C, ainsi qu'à différents agents chimiques.

## Revendications

1. Plateau prévu pour maintenir des tubes de dispositif d'injection (2) de médicaments comportant une collerette (6, 8) à chaque extrémité d'un corps (4), afin de réaliser des opérations de préparation de ces tubes (2), ce plateau (10) comprenant des perçages prévus pour recevoir chacun le corps du tube (4), **caractérisé en ce que** chaque perçage comporte une partie de maintien (16) configurée pour ne pas laisser passer la collerette supérieure (8) du tube (4), ladite partie de maintien (16) se prolongeant latéralement par une partie d'introduction (18) configurée pour permettre le passage de la collerette inférieure (6) du tube (4).

2. Plateau selon la revendication 1, **caractérisé en ce que** chaque perçage comporte d'un côté avant la partie de maintien (16) présentant vers l'avant un contour en arc de cercle prévu pour recevoir de manière ajustée le corps du tube (4).

3. Plateau selon la revendication 2, **caractérisé en ce que** la partie de maintien (16) se prolonge vers l'arrière par la partie d'introduction (18) comportant un contour en arc de cercle prévu pour permettre le passage de la collerette inférieure du tube (6).

4. Plateau selon la revendication 2 ou 3, **caractérisé en ce que** la partie de maintien (16) comporte vers l'avant une paroi verticale (22).

5. Plateau selon l'une quelconque des revendications précédentes, **caractérisé en ce qu'**il comporte des éléments élastiques (20) disposés entre la partie de maintien (16) et la partie d'introduction (18).

6. Plateau selon la revendication 5, **caractérisé en ce que** les éléments élastiques (20) forment des languettes comportant chacune un bossage séparant la partie de maintien (16) de la partie d'introduction (18).

7. Plateau selon l'une quelconque des revendications précédentes, **caractérisé en ce qu'**il comporte une matière plastique comprenant des polymères à cristaux liquides, mise en œuvre par injection.

## Patentansprüche

1. Platte, die zum Halten von Röhrchen einer Injektionsvorrichtung (2) von Arzneimitteln vorgesehen ist, die einen Kragen (6, 8) an jedem Ende eines Körpers (4) umfassen, um Vorgänge zur Vorbereitung dieser Röhrchen (2) durchzuführen, wobei diese Platte (10) Bohrungen umfasst, die jeweils zum Aufnehmen des Körpers des Röhrchens (4) vorgesehen sind, **dadurch gekennzeichnet, dass** jede Bohrung einen Halteteil (16) umfasst, der ausgestaltet ist, den oberen Kragen (8) des Röhrchens (4) nicht durchzulassen, wobei der Halteteil (16) seitlich von einem Einführteil (18) verlängert wird, der ausgestaltet ist, den unteren Kragen (6) des Röhrchens (4) durchzulassen.

2. Platte nach Anspruch 1, **dadurch gekennzeichnet, dass** jede Bohrung vorderseitig den Halteteil (16) umfasst, der nach vorne eine Kreisbogenkontur aufweist, die vorgesehen ist, um den Körper des Röhrchens (4) angepasst aufzunehmen.

3. Platte nach Anspruch 2, **dadurch gekennzeichnet, dass** der Halteteil (16) nach hinten von dem Einführteil (18) verlängert wird, der eine Kreisbogenkontur umfasst, die vorgesehen ist, um den unteren Kragen des Röhrchens (6) durchzulassen.

4. Platte nach Anspruch 2 oder 3, **dadurch gekennzeichnet, dass** der Halteteil (16) nach vorne eine vertikale Wand (22) umfasst.

5. Platte nach einem der vorstehenden Ansprüche, **dadurch gekennzeichnet, dass** sie elastische Elemente (20) umfasst, die zwischen dem Halteteil (16) und dem Einführteil (18) angeordnet sind.

6. Platte nach Anspruch 5, **dadurch gekennzeichnet, dass** die elastischen Elemente (20) Laschen bilden, die jeweils Ausbauchungen umfassen, die den Halteteil (16) von dem Einführteil (18) trennen.

7. Platte nach einem der vorstehenden Ansprüche, **dadurch gekennzeichnet, dass** sie einen Kunststoff umfasst, der flüssigkristalline Polymere, ausgeführt durch Einspritzung, aufweist.

## Claims

1. A tray intended to hold tubes of a device (2) for injecting drugs including a collar (6, 8) at each end of a body (4), in order to carry out operations of preparing these tubes (2), this tray (10) comprising holes each intended to receive the body of the tube (4), **characterized in that** each hole includes a holding portion (16) configured not to let the upper collar (8) of the tube (4) pass, said holding portion (16) extending laterally by an introduction portion (18) configured to enable the passage of the lower collar (6) of the tube (4).

2. The tray according to claim 1, **characterized in that** each hole includes at a front side the holding portion (16) having at the front a circle-arc like contour intended to adjustably receive the body of the tube (4).

3. The tray according to claim 2, **characterized in that** the holding portion (16) extends rearwards by the introduction portion (18) including a circle-arc like contour intended to enable the passage of the lower collar of the tube (6).

4. The tray according to claim 2 or 3, **characterized in that** the holding portion (16) includes at the font a vertical wall (22).

5. The tray according to any one of the preceding claims, **characterized in that** it includes elastic elements (20) disposed between the holding portion (16) and the introduction portion (18).

6. The tray according to claim 5, **characterized in that** the elastic elements (20) form tabs each including a boss separating the holding portion (16) from the introduction portion (18).

7. The tray according to any one of the preceding claims, **characterized in that** it includes a plastic material comprising liquid-crystal polymers, implemented by injection.
